# FASCICULE DE BREVET EUROPEEN

(11) **EP 1 089 673 B1**
(45) Date de publication et mention de la délivrance du brevet: **10.04.2002**
(21) Numéro de dépôt: 99957141.7
(22) Date de dépôt: 23.06.1999
(51) Int. Cl.: A61F 2/00, D04B 21/12

(54) **TISSU PROTHETIQUE ISOELASTIQUE AJOURE**
DURCHBROCHENE ISOELASTISCHE GEWEBEPROTHESE
ISOELASTIC PROSTHETIC FILET STITCH FABRIC

(30) Priorité: 23.06.1998 FR 9808106
(43) Date de publication de la demande: 11.04.2001
(73) Titulaire: SOFRADIM PRODUCTION, 01600 Trévoux (FR)
(72) Inventeur: ORY, Francois, Régis, F-69400 Fontaines Saint Martin (FR); THERIN, Michel, F-69002 Lyon (FR); MENEGHIN, Alfredo, F-69400 Villefranche sur Saone (FR)
(74) Mandataire: Guerre, Dominique
(86) Numéro de dépôt international: FR9901512
(87) Numéro de publication internationale: WO9966860

(56) Documents cités:
- WO-A-97/02789
- US-A- 3 124 136
- US-A- 5 456 711

## Description

La présente invention concerne un tricot isoélastique ajouré, utile en particulier en chirurgie pariétale et/ou viscérale, mais pouvant également trouver application en d'autres domaines de la chirurgie.

La présente invention sera plus particulièrement décrite par rapport à un tricot prothétique du type précité, destiné à être utilisé pour la réparation de hernies ou d'éventrations. La paroi abdominale est constituée de muscles déformables physiologiquement dans toutes les directions, et donc un renfort idéal doit suivre ces déformations physiologiques tout en offrant une résistance complémentaire à une paroi fragilisée. Même si les données publiées manquent, la limite inférieure de la résistance à la rupture d'un tricot prothétique pour la réparation de hernies ou d'éventrations se situe aux alentours de 10 kg, lorsque testée selon la norme NFG 07-001.

Dans la présente demande les expressions données ci-après ont la signification suivante :
- par "tissu", on entend un assemblage de fils, obtenu notamment par tricotage et/ou tissage ;
- par "matériau polymère biocompatible", on entend un matériau, ou ses éventuels produits de dégradation en milieu biologique, qui n'induit pas d'effet toxique ou contraire à l'effet recherché, une fois implanté dans le corps.

Depuis longtemps, les chirurgiens ont utilisé des tissus prothétiques plats, c'est-à-dire à deux dimensions, pour réparer ou remplacer toute partie du corps, telle qu'une aponévrose, ou une paroi musculaire, détruite ou endommagée, par exemple suite à un traumatisme. Par conséquent, il existe aujourd'hui un grand nombre de tels tissus prothétiques, fabriqués ou obtenus selon divers procédés, par exemple tissage, tricotage, ou moulage, et qui ont été conçus pour remplir souvent des fonctions spécifiques au sein du corps dans lequel ils sont implantés. De tels tissus prothétiques sont décrits par exemple dans les documents US-A-5569273, WO-A-96/03091 et EP-A-0797962, qui divulguent le tricotage de mono- ou multifilaments de polypropylène ou de polyester.

La demande de brevet PCT WO-A-97/02789, décrit un tricot tricoté ajouré à base d'un monofilament d'un matériau polymère biocompatible, l'armure dudit tissu déterminant une multiplicité d'alvéoles présentant une forme sensiblement polygonale. Le tissu est destiné notamment à la réparation de hernies, et est à base d'un monofilament de polypropylène, de 0,15 mm de diamètre, dont le motif de tricotage est défini par deux nappes de fils tricotés ensemble selon le barème suivant :
- nappe avant : 2/0-2/4-2/0-4/6-4/2-4/6;
- nappe arrière : 4/6-4/2-4/6-2/0-2/4-2/0.

Le but de l'invention décrite dans ce document est de fournir un tissu avec une meilleure transparence pour faciliter son utilisation dans les techniques de réparation laparoscopiques. Par ailleurs, il est indiqué, à la page 1, lignes 31-32 de la demande publiée, que le tricot doit présenter les caractéristiques physiques et performance des tissus prothétiques de réparation traditionnels.

Toutefois, de tels tissus traditionnels présentent plusieurs inconvénients, liés à la structure même du tissu, ou au choix du matériau de base utilisé pour constituer les fils, ou encore à la façon dont le tissu prothétique est fabriqué. Ainsi, les propriétés mécaniques des tissus traditionnels obtenus sont extrêmement variables d'un tissu à l'autre en fonction de l'orientation de l'échantillon: verticale (appelée également sens production ou colonnes) ou horizontale (appelée également sens travers ou rangées). A titre d'exemple, le barème de tricotage utilisé dans le document WO-A-97/02789 permet d'obtenir un tricot qui est environ deux fois plus résistant à la rupture en trame qu'en chaîne, ce qui correspond tout à fait aux propriétés des tissus traditionnels. Une récente publication compare les propriétés mécaniques des principaux tissus utilisés à ce jour (cf. Tableau 1 ci-après). Les écarts vont de 1 à 40 pour la résistance à la déchirure et de 1 à 8 pour la résistance à la rupture. Les valeurs d'allongement sous des charges physiologiques ou à la rupture sont rarement mentionnées (encore moins dans le sens diagonale), alors qu'elles constituent un paramètre important pour la fonctionnalité de ce type de produit.

**Tableau 1**

| | | Marlex® | Prolene® | Mersilene® |
|---|---|---|---|---|
| Fabricant | | Bard | Ethicon | Ethicon |
| Type | | monofilament PP | bifilament PP | multifilament PET |
| Poids/m² | | 95 | 108.5 | 39.5 |
| Résistance à la | verticale | 43 | 60 | 20 |
| rupture (daN) | horizontale | 57 | 77 | 10 |
| Résistance à la | verticale | 0.7 | <0.1 | 0.6 |
| déchirure (daN) | horizontale | 4 | 4.4 | 0.7 |

Source: U. Klinge & al: Veränderung der Bauchwandmechanik nach Mesh-lmplantation. Langenbecks Arch Chir (1996) 381:323-332

Par ailleurs, il est également connu que la réaction tissulaire à un implant est le résultat d'un processus normal de cicatrisation. Tout traumatisme chirurgical tissulaire est suivi d'une cascade d'événements physiologiques dont les principaux temps sont les suivants:
- t0 : traumatisme chirurgical, effraction capillaire ;
- t0 plus quelques minutes : coagulation, réseau fibrineux, libération facteurs chimiotactiques ;
- t0 plus 12 à 48h : afflux leucocytaire à dominante polynucléaire ;
- t0 plus 4 à 8 jours : afflux fibroblastique ;
- t0 plus 24h à 5 jours : afflux leucocytaire à dominante macrophagique ;
- t0 plus 8 à 21 jours : différenciation conjonctive de la réaction cicatricielle ;
- t0 plus 15 à 180 jours : remodelage cicatriciel au contact de l'implant.

Même si les mécanismes exacts sont pour certains encore inconnus, notamment en ce qui concerne le déterminisme de l'intensité de la réaction, il apparaît donc que les 8 premiers jours sont déterminants puisqu'ils conditionnent l'afflux fibroblastique.

En tissus non osseux, l'équilibre de la réaction conduit à la formation d'une membrane fibroconjonctive qui constitue l'interface entre le matériau implanté et le tissu sain environnant. Quelque soit le type d'implant, la zone sous influence direct d'un matériau conventionnel biocompatible est au minimum d'environ 50 micromètres.

Par ailleurs, dans le traitement des insuffisances pariétales (hernies et éventrations principalement), le tissu prothétique a pour vocation d'apporter un complément de résistance mécanique à la reconstruction chirurgicale, et est d'autant plus efficace, et sa tolérance locale d'autant meilleure, que son intégration tissulaire est intime et précoce.

La Demanderesse a établi par ses propres études que plusieurs aspects influencent la réponse tissulaire à un implant prothétique :
- le matériau constitutif de la prothèse et ses éventuels produits de dégradation en milieu biologique ne doivent pas induire d'effet toxique ou contraire à l'effet recherché. Dans le cas d'un tissu prothétique implanté à long terme, le vieillissement du matériau et ses conséquences (usure, relarguage de composants, etc...) est le facteur le plus difficile à anticiper ; seules des matières premières validées de longue date apportent un maximum de sécurité ;
- l'organisme ne voyant pour ainsi dire que la surface du matériau, les propriétés de celle-ci revêtent une importance non négligeable. Parmi l'ensemble des paramètres de surface, l'énergie de surface et la rugosité tiennent un rôle important. En effet, lorsque l'on recherche à promouvoir l'intégration cellulaire, la surface doit être hydrophile (énergie de surface élevée) et lisse à l'échelle d'une cellule (de l'ordre du micron) ;
- seule la porosité accessible par l'organisme est utile en ce qui concerne l'ancrage du tissu prothétique. Cette porosité dans un volume donné doit être interconnectée, et l'espace doit être suffisant pour une pénétration cellulaire significative (de l'ordre de 20 à 80 micromètres environ), et pour une différenciation tissulaire (100 à 300 micromètres constituent en général un minimum pour une différenciation complète). Il a été rappelé précédemment que l'épaisseur minimum de la réaction tissulaire est de l'ordre de 50 micromètres, ce qui signifie que pour des tailles de porosité inférieures à 100 micromètres, le tissu de réhabitation va entièrement être sous l'influence de la présence de l'implant avec peu de possibilité pour une différenciation tissulaire achevée de survenir ;
- la vascularisation et l'environnement biomécanique du site receveur conditionnent l'intensité de la réponse tissulaire. Un site richement vascularisé (peau, muscles, etc...) réagira plus vite et plus intensément que des tissus moins vascularisés (chambre antérieure de l'oeil, os, etc...). Par ailleurs, la nature même du site receveur conditionne la capacité de régénération à l'identique du tissu lésé. L'os, les tissus conjonctifs, les muqueuses, certains parenchymes (le foie par exemple) peuvent se régénérer à l'identique sans cicatrice fibreuse importante. En revanche d'autres tissus très spécialisés (muscles, tissus nerveux, etc...) ont perdu toute capacité de régénération, la cicatrisation de ces tissus ne se faisant alors que par fibrose ;
- le traumatisme chirurgical constitue l'un des principaux facteurs déclenchants de la réaction en cascade décrite précédemment. Plus celui-ci sera important, plus la réaction sera intense et ses conséquences prononcées (délai de cicatrisation, séquelles fibreuses, douleurs, etc...) ;
- le renfort est d'autant plus efficace et sa tolérance locale d'autant meilleure que son intégration tissulaire est intime et précoce. Pour être intime et précoce sans formation de coque fibreuse périphérique, les macroporosités de l'implant doivent le plus largement possible être ouvertes sur l'extérieur et l'élasticité du renfort doit lui permettre de suivre les déformations physiologiques de la paroi. Les limites supérieures étant données par la résistance mécanique (> 10 Kg en test normalisé type NFG07-001) du textile, la maniabilité par le chirurgien et l'impossibilité de récidive de hernie par les pores du tissu de 5 mm de diamètre maximum).

Compte tenu de ce qui précède, un des objectifs d'un bon tissu prothétique est une intégration tissulaire la plus rapide possible, procurant un ancrage mécaniquement satisfaisant sans fibrose extensive source d'inconfort et de douleur.

La présente invention décrit un nouveau type de renfort tricoté offrant des propriétés mécaniques élastiques isotropes, c'est à dire sensiblement équivalentes dans toutes les directions tout en préservant une porosité élevée pour une meilleure réhabitation tissulaire et une rigidité suffisante pour une maniabilité chirurgicale satisfaisante.

Le tricot concerné est un tricot prothétique isoélastique ajouré à base d'un monofilament d'un matériau polymère biocompatible, dont l'armure est définie par une nappe avant et une nappe arrière de fils tricotées ensemble et dont l'armure détermine une multiplicité d'alvéoles présentant chacune une forme sensiblement polygonale.

Selon l'invention, ladite armure confère au tricot un comportement dynamométrique multidirectionnel et est obtenue par une nappe avant obtenue par tricotage selon un barème 5-4/4-3/2-1/0-1/1-2/3-4 et par une nappe arrière obtenue par tricotage selon un barème 0-1/1-2/3-4/5-4/4-3/2-1.

Le déposant a découvert qu'un tricot prothétique à base d'un monofilament biocompatible tel que précédemment défini présente les avantages suivants :
- le tricot est souple et peu dense en matériau, le poids au m² étant réduit, par exemple d'au moins 30%, par rapport aux produits équivalent sur le marché, ce qui permet d'obtenir une meilleure tolérance locale ;
- le tricot présente une tenue suffisante (effet ressort lors des déformations imposées) pour une maniabilité aisée par le chirurgien ;
- le tricot est indémaillable quelques soient les découpes pratiquées par le chirurgien, avec de faibles chutes de filaments le long de la ligne de coupe, contrairement aux tissus connus ;
- l'isoélasticité, illustrée par des pentes relativement linéaires de courbes force/déformation en fonction du sens de traction du tricot, permet d'obtenir une adéquation biomécanique avec la paroi abdominale ;
- le tricot est relativement transparent pour une meilleure visibilité des structures anatomiques couvertes notamment lors de mise en place laparoscopique ;
- le tricot est poreux avec des jours principaux de l'ordre du millimètre pour une meilleure pénétration cellulaire et une différenciation conjonctive à coeur sans phénomène de coque fibreuse périphérique source potentielle d'inconfort et de douleur.

Dans un mode d'exécution préféré de l'invention, le tricot présente un comportement dynamométrique défini par un rapport de valeurs mesurables, par exemple de résistance à la rupture, dans les directions verticale/horizontale/diagonale de contrainte compris entre 0,9:1:1,5 à 1,5:1:1,5.

De préférence, le tricot présente au moins l'une quelconque des propriétés suivantes :
- la force à la rupture du tricot selon la norme NFG07-001 est de l'ordre de 25 daN dans toutes les directions ;
- l'allongement à la rupture du tricot selon la norme NFG07-001 est de l'ordre de 70 % dans toutes les directions ;
- la résistance à la déchirure amorcée du tricot selon la norme NFG07-001 est de l'ordre de 5 daN ;
- l'allongement sous contrainte de 10 daN du tricot mésure avec une tension initiale modérée de l'ordre de 2 daN est de l'ordre de 35 % dans toutes les directions.

De préférence, le tricot ajouré est à base d'un monofilament d'un matériau polymère biocompatible choisi dans le groupe consistant en le polypropylène, le polyester, et le polyamide, et de préférence est un monofilament de polypropylène.

Préférentiellement, le diamètre du monofilament est compris entre 0,12 mm et 0,18 mm, et de préférence est de 0,15 mm.

De préférence, l'armure du tricot forme des mailles ouvertes non bloquées définissant des alvéoles principales entourées par des alvéoles de plus petites dimensions, favorisant la réhabitation.

Avantageusement, chaque alvéole principale présente un diamètre d'environ 1 mm à environ 2 mm, et de préférence un diamètre compris entre 1,5 mm et 1,9 mm, tandis que les alvéoles de plus petites dimensions ont un diamètre compris entre 0,4 et 0,8 mm et de péférence entre 0,6 et 0,7.

L'épaisseur du tricot est comprise entre 0,5 mm et 1 mm, et de préférence est au plus égale à 0,7 mm.

De manière préférée, le tricot présente un poids surfacique compris entre 50 g/m² et 100 g/m², et de préférence un poids spécifique d'environ 75°g/m².

Un autre objet de la présente invention est une prothèse pariétale et/ou viscérale, obtenue avec un tricot tel que défini précédemment.

Encore un autre objet de la présente invention est l'utilisation d'un tricot selon l'invention tel que défini, pour obtenir un produit prothétique à usage chirurgical, notamment pour fabriquer une prothèse pariétale et/ou viscérale.

La présente invention sera mieux comprise par rapport à la description détaillée d'un mode d'exécution préféré de l'invention, donnée à titre d'exemple, en association avec les figures en annexe, dans lesquelles :
- la **Figure 1** représente une image de face d'un tricot prothétique isoélastique ajouré conformément à l'invention, prise par microscopie électronique à balayage avec un agrandissement de 20 fois ;
- la **Figure 2a** représente un dessin schématique simplifié d'un fil avant (AV) et d'un fil arrière (AR) de l'armure de tricotage du tricot prothétique isoélastique selon la figure 1 ;
- la **Figure 2b** représente un dessin schématique simplifié de l'armure de tricotage du tricot prothétique isoélastique selon la figure 1 ;
- la **Figure 3a** représente un dessin schématique simplifié d'un fil avant (AV) et d'un fil arrière (AR) de l'armure de tricotage d'un tricot prothétique selon l'art antérieur ;
- la **Figure 3b** représente un dessin schématique simplifié de l'armure de tricotage d'un tricot prothétique selon l'art antérieur ;
- la **Figure 4** représente un graphique de courbes force/déformation en fonction du sens de traction du tricot isoélastique de la Figure 1 ;
- la **Figure 5** représente un graphique de courbes force/déformation d'un tricot ajouré isoélastique selon la Figure 1, sous une précontrainte de 2 daN.

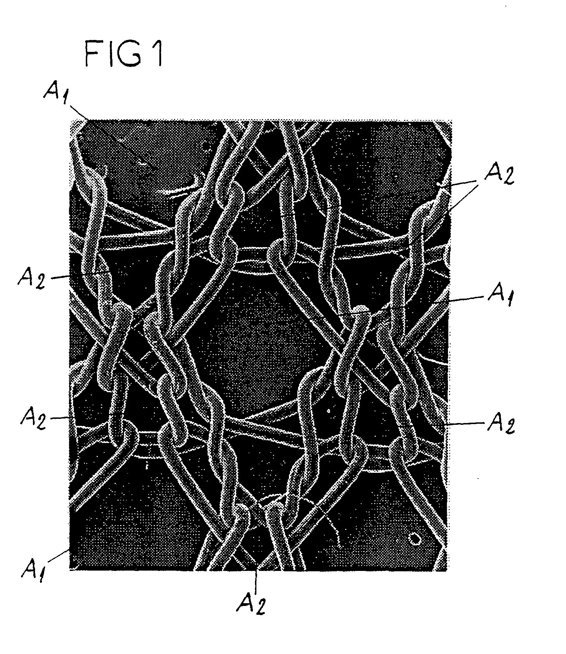

En se référant à la Figure 1, le tricot selon la présente invention est un tricot ajouré, en monofilament de polypropylène, de 0,15 mm de diamètre, réalisé sur métier chaîne ou métier rachel avec deux nappes ou barres à passettes enfilées un plein, un vide, se déplaçant symétriquement. Le motif est un atlas mailles ouvertes avec des progressions sous deux et trois aiguilles. Cela permet d'obtenir des valeurs similaires d'élasticité horizontale et d'élasticité horizontale et diagonale. On obtient ainsi un tricot aux caractéristiques dynamométriques multidirectionnelles relativement équilibrées.

La figure 1 montre bien que chaque alvéole principale A1 est entourée par des alvéoles périphériques A2 de plus petites dimensions aérant le tricot, augmentant sa porosité et supprimant tout amas local de fils, et améliorant sa transparence et les possibilités de réhabitation.

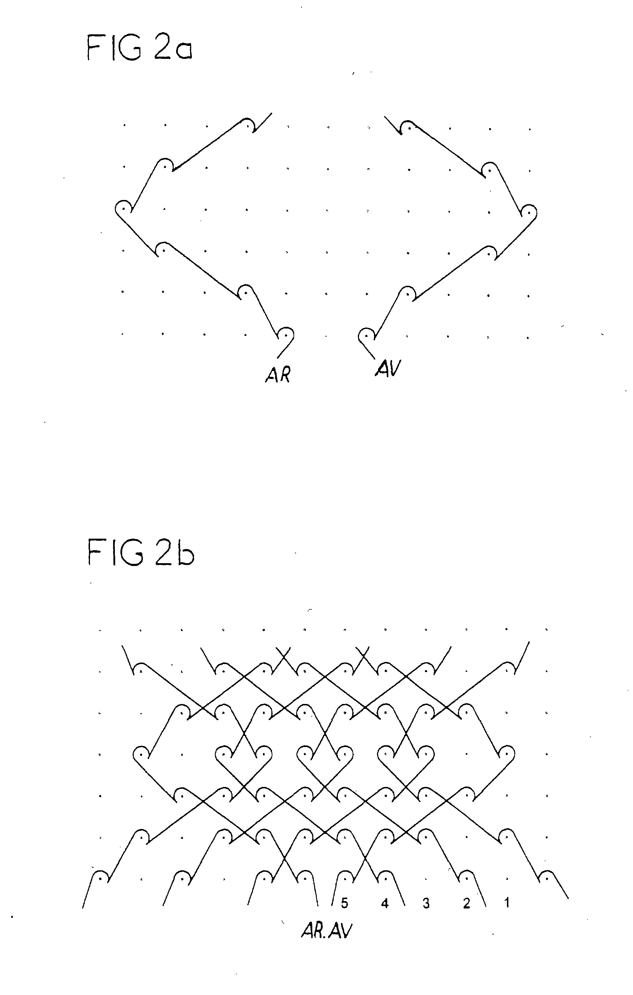

Le barème de tricotage est représenté schématiquement par les figures 2a et 2b, et est le suivant, à savoir :
- pour la nappe arrière, 0-1/1-2/3-4/5-4/4-3/2-1;
- pour la nappe avant: 5-4/4-3/2-1/0-1/1-2/3-4.

La figure 2a indique seulement un fil avant (AV) et un fil arrière (AR) de l'armure pour montrer plus clairement les déplacements des fils. Ainsi on constate que les fils du tricot selon l'invention se déplacent au total sous cinq aiguilles, ce qui permet d'obtenir une meilleure tenue des fils, avec moins de risques d'effilochage des bords, et de démaillage.

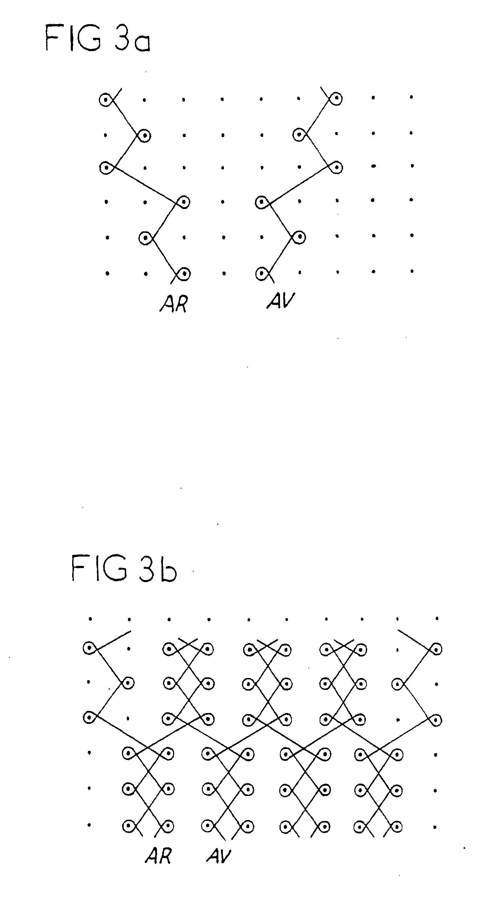

Les figures 3a et 3b illustrent le schéma de l'armure utilisé pour obtenir un tricot prothétique selon la demande WO-A-97/02789, et permettent de voir les différences avec le tricot isoélastique selon l'invention par comparaison avec les figures 2a et 2b. Ainsi, on constate que les fils du tricot de l'art antérieur se déplacent sous trois aiguilles et non cinq comme dans le tricot de la présente invention, ce qui conduit notamment à des chutes plus importantes de filaments lors de la recoupe du tricot par le chirurgien, comparées au tricot selon l'invention.

Par ailleurs, l'utilisation de mailles ouvertes dans le tricot selon l'invention par rapport aux mailles fermées du tricot de la demande WO-A-97/02789 (cf. figures 3a et 3b) permet plus de liberté dans le tricotage, et donc de mieux contrôler son élasticité.

Des exemples de résultats pour un tricot réalisé à partir de monofil de polypropylène de diamètre 0,15 mm, thermofixé en traction horizontale à 150°C (poids au m2 : environ 75 g), sont donnés dans le Tableau 2 ci-après.

**Tableau 2**

| | **Verticale** | **Horizontale** | **Diagonale** |
|---|---|---|---|
| **Force à la rupture (daN) norme NFG07-001** | 31 | 29 | 29 |
| **Allongement à la rupture (%) norme NFG07-001** | 70 | 107 | 68 |
| **Allongement sous 10 daN (%) norme NFG07-001** | 40 | 67 | 31 |
| **Résistance à la déchirure amorcée (daN) norme NFG07-149** | 5.6 | 5.1 | - |
| **Eclatométrie - norme NFG07-112** | | | |
| **- Pression en Mpa** | | 0.82 | |
| **- Flèche en mm** | | 14.4 | |

On constate que quelque soit l'orientation de l'échantillon testé, les résistances à la rupture et à la déchirure amorcée sont relativement homogènes. Avec le type de fil utilisé pour cet essai, la résistance à la rupture est nettement supérieure aux valeurs minimales requises par ce genre de renfort (10 à 15 daN). La résistance à la déchirure amorcée constitue un paramètre important qui illustre la solidité des ancrages aux points de fixation ou lors de sollicitations sur des renforts aux contours découpés et complexes. Cette résistance à la déchirure est très nettement supérieure à celles mesurées pour les produits équivalents actuellement sur le marché (cf. Tableau 1).

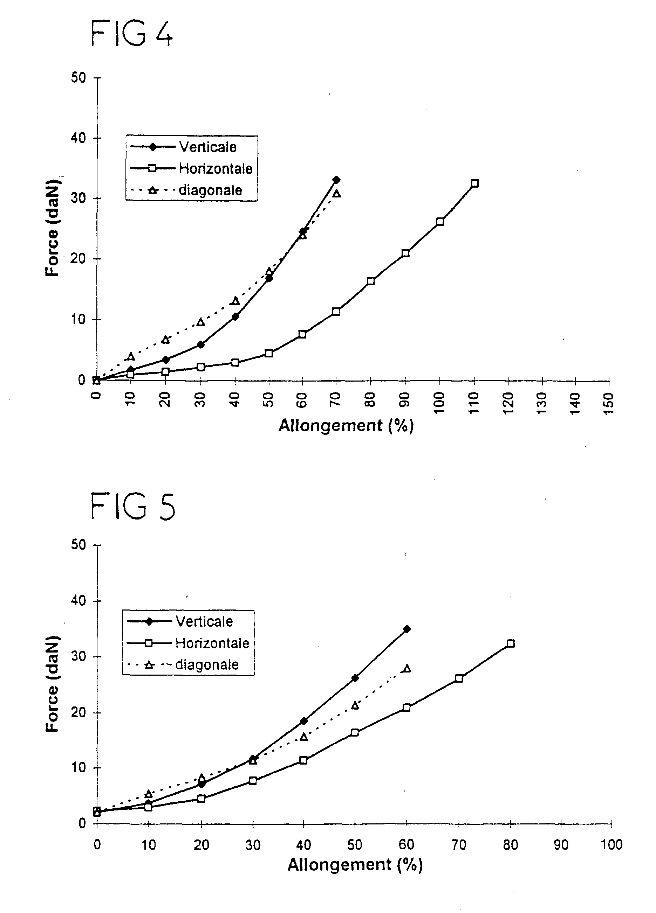

La figure 4 montre qu'à contrainte équivalente, les directions verticale et diagonale ont quant à elles des courbes pratiquement superposées. Si l'on considère qu'en chirurgie ouverte et plus particulièrement dans le cas du traitement des éventrations, les renforts sont implantés avec une tension initiale modérée pouvant être estimée de l'ordre de 2daN, alors les courbes force/déformation en fonction de la direction de traction deviennent très proches les unes des autres (cf. Figure 5). L'élasticité modérément plus importante selon la direction horizontale peut donner une latitude au chirurgien quant à la direction privilégiée des déformations autorisées. En effet, beaucoup de chirurgiens considèrent que les besoins en élasticité sont supérieurs dans le sens supéro-inférieur, car il y a une flexion fréquente de la colonne vertébrale par contraction des muscles grand droits de l'abdomen, que dans la direction latéro-latérale, qui correspond plutôt à une distension progressive de la sangle abdominale. Le tricot selon la présente invention permet d'accommoder de manière suffisante cette préférence chirurgicale, sans toutefois perdre les propriétés mécaniques avantageuses dans les autres directions de traction.

## Revendications

1. Tricot prothétique isoélastique ajouré à base d'un monofilament d'un matériau polymère biocompatible, dont l'armure est définie par une nappe avant et une nappe arrière de fils tricotées ensemble et détermine une multiplicité d'alvéoles présentant chacune une forme sensiblement polygonale, ladite armure confèrant au tricot un comportement dynamométrique multidirectionnel **caractérisé en ce que** ladite armure est obtenue par une nappe avant obtenue par tricotage selon un barème 5-4/4-3/2-1/0-1/1-2/3-4 et par une nappe arrière obtenue par tricotage selon un barème 0-1/1-2/3-4/5-4/4-3/2-1.

2. Tricot selon la revendication 1, **caractérisé en ce que** son armure forme des mailles ouvertes non bloquées définissant des alvéoles principales (A1) et des alvéoles périphériques (A2) entourant ces dernières mais de plus petites dimensions.

3. Tricot selon la revendication 2, **caractérisé en ce que** chaque alvéole (A1) présente un diamètre d'environ 1 mm à environ 2 mm, tandis que les alvéoles périphériques (A2) ont un diamètre compris entre 0,4 et 0,8 mm.

4. Tricot selon la revendication 1, **caractérisé en ce que** son épaisseur est comprise entre 0,5 mm et 1 mm.

5. Tricot selon la revendication 4, **caractérisé en ce que** son épaisseur est au plus égale à 0,7 mm.

6. Tricot selon la revendication 1, **caractérisé en ce que** sa force à la rupture selon la norme NFG07-001 est de l'ordre de 25 daN dans toutes les directions.

7. Tricot selon la revendication 1, **caractérisé en ce que** son allongement à la rupture, mesuré avec une tension initiale modérée, de l'ordre de 2 daN, est de l'ordre de 80 % dans toutes les directions.

8. Tricot selon la revendication 1, **caractérisé en ce que** sa résistance à la déchirure amorcée selon la norme NFG07-001 est de l'ordre de 5 daN.

9. Tricot selon la revendication 1, **caractérisé en ce que** son allongement sous contrainte de 10 daN, mesuré avec une tension initiale modérée, de l'ordre de 2 daN, est de l'ordre de 35 % dans toutes les directions.

10. Tricot selon la revendication 1, **caractérisé en ce qu'**il présente un poids surfacique compris entre 50 g/m2 et 100 g/m².

11. Tricot selon la revendication 10, **caractérisé en ce qu'**il présente un poids spécifique d'environ 75 g/m².

12. Tricot selon la revendication 1, **caractérisé en ce qu'**il est réalisé sur métier chaîne ou métier rachel avec deux nappes ou barres à passettes enfilées un plein, un vide, se déplaçant symétriquement, le motif étant un atlas mailles ouvertes avec des progressions sous deux et trois aiguilles.

13. Prothèse pariétale et/ou viscérale, obtenue avec un tricot selon l'une quelconque des revendications 1 à 12.

## Claims

1. Open-worked isoelastic prosthetic knitted fabric based on a monofilament of a biocompatible polymer material, the weave of which is defined by a front lap and a rear lap of threads which are knitted together, and defines a multiplicity of alveoles, each of which has a substantially polygonal shape, the said weave providing the knitted fabric with multi-directional dynamometric behaviour, **characterised in that** the said weave is obtained by means of a front lap which is obtained by knitting according to a scale of 5-4/4-3/2-1/0-1/1-2/3-4, and by means of a rear lap which is obtained by knitting according to a scale of 0-1/1-2/3-4/5-4/4-3/2-1.

2. Knitted fabric according to claim 1, **characterised in that** its weave forms unblocked open meshwork, which defines main alveoles (A1) and peripheral alveoles (A2) which surround the latter, but have smaller dimensions.

3. Knitted fabric according to claim 2, **characterised in that** each alveole (A1) has a diameter of approximately 1 mm to approximately 2 mm, whereas the peripheral alveoles (A2) have a diameter of between 0.4 and 0.8 mm.

4. Knitted fabric according to claim 1, **characterised in that** its thickness is between 0.5 mm and 1 mm.

5. Knitted fabric according to claim 4, **characterised in that** its thickness is 0.7 mm at the most.

6. Knitted fabric according to claim 1, **characterised in that** its breaking load according to standard NFG07-001 is approximately 25 daN in all directions.

7. Knitted fabric according to claim 1, **characterised in that** its breaking elongation, measured with a moderate initial tension of approximately 2 daN, is approximately 80% in all directions.

8. Knitted fabric according to claim 1, **characterised in that** its resistance to tear propagation according to standard NFG07-001 is approximately 5 daN.

9. Knitted fabric according to claim 1, **characterised in that** its elongation under a force of 10 daN, measured with a moderate initial tension of approximately 2 daN, is approximately 35% in all directions.

10. Knitted fabric according to claim 1, **characterised in that** it has a surface weight of between 50 g/m² and 100 g/m².

11. Knitted fabric according to claim 10 **characterised in that** it has a specific weight of approximately 75 g/m².

12. Knitted fabric according to claim 1, **characterised in that** it is produced on a warp loom or a Raschel loom with two laps or guide bars, which produce a solid part and a gap in succession, and are displaced symmetrically, the pattern being an open meshwork atlas with progressions using two and three needles.

13. Parietal and/or visceral prosthesis, obtained by means of a knitted fabric according to any one of claims 1 to 12.

## Patentansprüche

1. Durchbrochenes isoelastisches prothetisches Gewirk auf der Basis eines Monofilaments aus einem biokompatiblen polymeren Material, dessen Bindung durch eine vordere Schar und eine hintere Schar von zusammen gewirkten Fäden definiert ist und eine Vielzahl von Alveolen bestimmt, die jeweils eine etwa polygonale Form aufweisen, wobei die Bindung dem Gewirk ein vielgerichtetes dynamometrisches Verhalten verleiht, **dadurch gekennzeichnet, daß** die Bindung durch eine vordere Schar, die durch Wirken gemäß einem Schema 5-4/4-3/2-1/0-1/1-2/3-4 hergestellt ist, und durch eine hintere Schar hergestellt ist, die durch Wirken gemäß einem Schema 0-1/1-2/3-4/5-4/4-3/2-1 hergestellt ist.

2. Gewirk nach Anspruch 1, **dadurch gekennzeichnet, daß** seine Bindung nicht fixierte offene Maschen bildet, die Hauptalveolen (A1) und periphere Alveolen (A2) definieren, die letztere umgeben, jedoch kleinere Abmessungen aufweisen.

3. Gewirk nach Anspruch 2, **dadurch gekennzeichnet, daß** jede Alveole (A1) einen Durchmesser von etwa 1 mm bis etwa 2 mm aufweist, während die peripheren Alveolen (A2) einen Durchmesser im Bereich von 0,4 bis 0,8 mm aufweisen.

4. Gewirk nach Anspruch 1, **dadurch gekennzeichnet, daß** seine Dicke im Bereich von 0,5 mm bis 1 mm liegt.

5. Gewirk nach Anspruch 4, **dadurch gekennzeichnet, daß** seine Dicke höchstens gleich 0,7 mm beträgt.

6. Gewirk nach Anspruch 1, **dadurch gekennzeichnet, daß** seine Bruchkraft gemäß der Norm NFG07-001 in allen Richtungen in der Größenordnung von 25 daN liegt.

7. Gewirk nach Anspruch 1, **dadurch gekennzeichnet, daß** seine Bruchdehnung, gemessen bei einer mäßigen anfänglichen Spannung in der Größenordnung von 2 daN, in allen Richtungen in der Größenordnung von 80 % liegt.

8. Gewirk nach Anspruch 1, **dadurch gekennzeichnet, daß** sein Weiterreißwiderstand gemäß der Norm NFG07-001 in der Größenordnung von 5 daN liegt.

9. Gewirk nach Anspruch 1, **dadurch gekennzeichnet, daß** seine Dehnung unter einer Beanspruchung von 10 daN, gemessen bei einer mäßigen anfänglichen Spannung in der Größenordnung von 2daN, in allen Richtungen in der Größenordnung von 35 % liegt.

10. Gewirk nach Anspruch 1, **dadurch gekennzeichnet, daß** es ein flächenbezogenes Gewicht im Bereich von 50 g/m² bis 100 g/m² aufweist.

11. Gewirk nach Anspruch 10, **dadurch gekennzeichnet, daß** es ein spezifisches Gewicht von etwa 75 g/m² aufweist.

12. Gewirk nach Anspruch 1, **dadurch gekennzeichnet, daß** es auf einem Kettstuhl oder einer Kachel-Maschine mit zwei Legescharen oder -barren gefertigt wird, die 'eines voll, eines leer' eingefädelt sind, die sich symmetrisch verschieben, wobei die Grundlegung ein Offen-Maschen-Atlas mit Progressionen unter zwei und drei Nadeln ist.

13. Parietale und/oder viszerale Prothese, die mit einem Gewirk nach einem der Ansprüche 1 bis 12 hergestellt ist.
